**Europäisches Patentamt**

**European Patent Office**

**Office européen des brevets**

(19)

(11) Veröffentlichungsnummer : **0 031 504**
**B1**

(12) **EUROPÄISCHE PATENTSCHRIFT**

(45) Veröffentlichungstag der Patentschrift :
15.12.82

(21) Anmeldenummer : **80107662.1**

(22) Anmeldetag : **05.12.80**

(51) Int. Cl.³ : **C 07 C 51/60**

(54) **Verfahren zur Herstellung von Carbonsäurechloriden.**

(30) Priorität : **13.12.79 DE 2950155**

(43) Veröffentlichungstag der Anmeldung :
**08.07.81 (Patentblatt 81/27)**

(45) Bekanntmachung des Hinweises auf die Patenterteilung : **15.12.82 Patentblatt 82/50**

(84) Benannte Vertragsstaaten :
**BE DE FR**

(56) Entgegenhaltungen :
**DE A 2 057 956**
**US A 3 149 155**

(73) Patentinhaber : **BASF Aktiengesellschaft**
**Carl-Bosch-Strasse 38**
**D-6700 Ludwigshafen (DE)**

(72) Erfinder : **Decker, Martin, Dr.**
**Maria-Stuart-Strasse 21**
**D-6700 Ludwigshafen (DE)**
Erfinder : **Merkel, Karl, Dr.**
**Sperlinggasse 15**
**D-6700 Ludwigshafen (DE)**
Erfinder : **Neumayr, Franz, Dr.**
**Im Elertal 8**
**D-6719 Weisenheim (DE)**

EP 0 031 504 B1

## Verfahren zur Herstellung von Carbonsäurechloriden

Diese Erfindung betrifft ein Verfahren zur Herstellung von Carbonsäurechloriden durch Umsetzung von Carbonsäuren oder deren Anhydriden mit Phosgen in Gegenwart von Diisobutylformamid.

Aus der BE-PS 620 385 und der DE-OS 20 57 956 ist bekannt, daß man Carbonsäuren durch Umsetzung mit Phosgen in die entsprechenden Carbonsäurechloride überführen kann, wenn man die Umsetzung in Gegenwart von Dialkylcarbonsäureamiden als Katalysatoren durchführt. Die technische Herstellung der Carbonsäurechloride nach diesen Verfahren bereitet Schwierigkeiten. Wird z.B. mit Dimethylformamid gearbeitet, so bleibt dieser Katalysator in der Regel nur solange im Gemisch gelöst, wie die Hauptmenge der Carbonsäure noch nicht mit Phosgen unter Bildung des Säurechlorids reagiert hat. Sobald die Konzentration an Phosgen im Verlauf der Umsetzung auf einige Prozent ansteigt, setzt sich der Katalysator als zähe Flüssigkeit am Boden des Reaktionsgefäßes ab und muß vom Säurechlorid abgetrennt werden. Diese Abtrennung ist sehr arbeitsintensiv.

Da man die Säurechloride bis zur Molekülgröße von etwa 10 C-Atomen in der Regel destillativ aufarbeitet, reichert sich der Katalysator bei ungenügender Abtrennung im Destillationssumpf an, wo er sich nach und nach zersetzt, wodurch das Destillat verunreinigt wird. Auch kann sich der Destillationssumpf bei Anwesenheit größerer Mengen an Dialkylformamidiniumchlorid in der Hitze spontan zersetzen.

Werden nicht destillierbare Säurechloride hergestellt, die z.B. mit Aktivkohle gereinigt werden, so tritt bei ungenügender Katalysatorabtrennung die Gefahr der Verstopfung der Leitungen zum Filter und des Filters auf, da der Katalysator bei Raumtemperatur fest wird. Unterbrechungen im Arbeitsablauf und Reinigungsarbeiten sind die Folge.

Bei der kontinuierlichen Herstellung von Säurechloriden treten die genannten Probleme im Vergleich zur diskontinuierlichen Herstellung in noch höherem Maße auf, wenn man Dialkylformamide einsetzt, die im Endprodukt unlöslich sind. Durch allmähliche Veränderung des Verhältnisses von Phosgen zu Carbonsäure kommt es immer wieder zu Ablagerungen von Katalysator in Leitungen und Wärmetauschern, weshalb die Anlage häufig abgestellt und gereinigt werden muß. Auch bei korrektem Zulaufverhältnis werden kleine Mengen des Katalysators mitgeschleppt und lagern sich nach und nach in Teilen der Apparatur, wie Ventilen, Pumpen und Leitungen ab. Hierdurch können mechanische Schäden an den Pumpen verursacht werden.

Nach den Angaben der BE-PS 620 385 lassen sich zwar die störenden Ablagerungen vermeiden, wenn man in der Reaktionszone die Carbonsäure nur unvollständig umsetzt. Jedoch sind im Endprodukt dann sowohl nicht umgesetzte Carbonsäure als auch das entsprechende Carbonsäureanhydrid enthalten, von denen das Säurechlorid abgetrennt werden muß.

Werden andere Formamide, wie Diäthylformamid, Di-n-propylformamid oder Di-isopropylformamid eingesetzt, so ergeben sich ähnliche Schwierigkeiten, da auch diese Katalysatoren in Form ihrer Formamidinidumchloride zu unlöslichen Ablagerungen neigen. Verwendet man Di-n-butylformamid oder Alkylformamide mit noch höheren Alkylresten, wie Di-2-ethylhexylformamid, so lassen sich zwar Ablagerungen der genannten Art vermeiden, man erhält dann jedoch bei der Destillation der Säurechloride verfärbte Produkte.

Es wurde nun überraschend gefunden, daß man bei der Herstellung von Säurechloriden durch Umsetzung von Carbonsäuren oder deren Anhydriden mit Phosgen in Gegenwart eines Dialkylformamids die genannten Nachteile vermeidet, wenn man als Dialkylformamid Diisobutylformamid verwendet.

Überraschenderweise erfüllt die erfindungsgemäße Verwendung des Diisobutylformamids in einzigartiger Weise die hohen Anforderungen bei der technischen Herstellung von Carbonsäurechloriden aus Carbonsäuren und Phosgen. Sowohl bei der diskontinuierlichen wie auch bei der kontinuierlichen Arbeitsweise werden bei dem erfindungsgemäßen Verfahren weder störende Ablagerungen noch Verfärbungen bei der destillativen Aufarbeitung der Carbonsäurechloride beobachtet.

Bevorzugte Ausgangsstoffe sind aliphatische Carbonsäuren oder deren Anhydride. Beispielsweise geht man von Carbonsäuren der allgemeinen Formel R—COOH aus, in der R ein Alkylrest mit 1 bis 18, vorzugsweise 1 bis 6 Kohlenstoffatomen bedeutet. Der Alkylrest kann noch durch unter den Reaktionsbedingungen inerte Gruppen und/oder Atome, z.B. Chloratome, Nitrogruppen, Alkylgruppen mit 1 bis 4 Kohlenstoffatomen, substituiert sein. Folgende Ausgangsstoffe sind beispielsweise geeignet: Essigsäure, Mono-, Di-, Tri-chloressigsäure, Propionsäure, Buttersäure, Valeriansäure, Laurylsäure, Stearinsäure, Capronsäure, Isovaleriansäure, Palmitinsäure, $\alpha$-Nitropropionsäure, 2-Äthylhexancarbonsäure.

Die aliphatische Carbonsäure wird mit der stöchiometrischen Menge oder zweckmäßig mit einem Überschuß an Phosgen, vorzugsweise in einem Verhältnis von 1,1 bis 1,5 Mol Phosgen je Mol Ausgangssäure, umgesetzt. Das Diisobutylformamid wendet man in einer Menge von 0,1 bis 0,4, vorzugsweise 0,15 bis 0,3 Mol-%, bezogen auf die Carbonsäure, an. Die Umsetzung wird bei Temperaturen von 30 bis 120 °C, vorzugsweise 50 bis 80 °C, drucklos oder unter Druck und diskontinuierlich oder kontinuierlich durchgeführt.

Die Umsetzung wird bei der absatzweisen Herstellung von Säurechloriden beispielsweise so durchgeführt, daß man die flüssige oder geschmolzene Carbonsäure in einem geeigneten Rührbehälter vorlegt, das Diisobutylformamid darin löst und das Gemisch auf die gewünschte Reaktionstemperatur erwärmt. Bereits während des Aufheizens kann mit dem Einleiten von Kohleoxichlorid begonnen werden.

2

Sobald die stöchiometrische Menge Kohlenoxichlorid eindosiert ist, wird die pro Zeiteinheit zugeführte Menge auf 10 bis 30 % der ursprünglichen Menge zurückgenommen. Durch starken Kohlenoxichlorid-Rückfluß wird das Ende der Reaktion angezeigt. Das Rohprodukt wird destillativ aufgearbeitet, wobei der Vorlauf des Carbonsäurechlorids das im Überschuß eingesetzte Kohlenoxichlorid enthält. Dieser wird beim nachfolgenden Ansatz mit der vorgelegten Carbonsäure nach und nach zur Reaktion gebracht, sobald mit der Zufuhr von Kohlenoxichlorid begonnen wird.

Bei der kontinuierlichen Arbeitsweise wird zunächst der Reaktor mit Carbonsäure und Katalysator bis nahe an den Überlauf gefüllt und bei der gewünschten Reaktionstemperatur mit Kohlenoxichlorid umgesetzt. Danach wird die Carbonsäure, Diisobutylformamid und Kohlenoxichlorid gleichzeitig und kontinuierlich dem Reaktor zugeführt. Der stündliche Volumenstrom der Carbonsäure, der im allgemeinen 10 bis 15 % des Reaktorvolumens beträgt, kann, besonders bei höhermolekularen Carbonsäuren, auf 40 bis 50 % des Reaktorvolumens erhöht werden, wenn die nachfolgende Aufarbeitung für diese Menge geeignet ist.

Das Dibutylformamid wird kontinuierlich hinzugefügt. Es kann auch vorab in der Carbonsäure gelöst und mit dieser zusammen dem Reaktor zugeführt werden.

Das Kohlenoxichlorid kann bei der kontinuierlichen Arbeitsweise in der Menge auf wenige Mol-% über die stöchiometrische Menge begrenzt werden, weil in einer dem Reaktor nachgeschalteten Kolonne das überschüssige Kohlenoxichlorid abgetrennt und sofort dem Reaktor wieder zugeführt wird. Der erforderliche Überschuß ist im wesentlichen von der Kühlleistung der Reaktionskühler abhängig.

Die nach dem Verfahren der Erfindung herstellbaren Verbindungen sind zum Teil Lösungsmittel und wertvolle Ausgangsstoffe für die Herstellung von Lösungsmittel, Riechstoffen, Weichmachern, Waschmitteln, Beizen und Schädlingsbekämpfungsmitteln. Bezüglich der Verwendung wird auf die genannten Veröffentlichungen und auf Ullmanns Encyklopädie der technischen Chemie, Band 5, Seite 100 ff. und 393 ff. verwiesen.

## Beispiel 1-7

In einem Rührbehälter mit 500 Volumenteilen Fassungsvermögen, der mit einem Rückflußkühler und Zulaufeinrichtungen ausgestattet ist, werden 163 Gewichtsteile Äthylhexansäurechlorid mit 0,2 Mol-% gelöstem Katalysator vorgelegt und auf 65 °C erwärmt. Die Temperatur des Kühlmittels im Rückflußkühler beträgt − 70 °C. In die vorgelegte Mischung werden stündlich 144 Gewichtsteile 2-Äthylhexansäure, in welcher 0,2 Mol-% Diisobutylformamid gelöst sind und 120 Gewichtsteile Kohlenoxidchlorid eingetragen. Aus einem Überlauf im oberen Bereich des Rührbehälters gelangt das rohe Reaktionsprodukt in einen Vorratsbehälter, aus welchem es der weiteren Verarbeitung zugeführt wird.

In analoger Weise werden die in der Tabelle genannten Säurechloride unter den dort angegebenen Bedingungen hergestellt. Sofern das im Überschuß eingesetzte Kohlenoxichlorid in der Aufarbeitungsstufe kondensiert und wieder der Reaktionsstufe zugeführt wird, erniedrigt sich die Zufuhr von frischem Kohlenoxichlorid nahezu auf die stöchiometrische Menge.

In der Verarbeitungsstufe wird das Reaktionsprodukt, sofern die Flüchtigkeit des Produktes es ermöglicht, destillativ gereinigt. Bei schwerflüchtigen Verbindungen kann die Reinigung mit Aktivkohle erfolgen. Wegen der guten Qualität der Rohprodukte erübrigt sich jedoch in vielen Fällen eine weitere Reinigung

### TABELLE

| Beispiel | Carbonsäure | Zulauf Säure [Gew.-Teile/h][2] | Zulauf COCl$_2$ [Gew.-Teile/h][2] | Versuchs- dauer [h] | Reaktions- temperatur [°C] | Rohprodukt- austräge Ausbeute [Mol-%] | Reinheit [Gew.-%] Säure- chlorid | Reinheit [Gew.-%] Säure bzw. Säure- anhydrid |
|---|---|---|---|---|---|---|---|---|
| 1 | 2-Äthylhexansäure | 144 | 120 | 46 | 62-65 | 98,8 | 98,9 | 0,42 |
| 2 | Methoxiessigsäure | 180 | 240 | 8 | 58-62 | 97,7 | 99,4 | 0,32 |
| 3 | Palmitinsäure | 256 | 115 | 12 | 55-65 | 98,9 | 99,3 | 0,15 |
| 4 | Azelaninsäure | 105 | 130 | 9 | 70-95 | 99,0 | 99,2 | 0 |
| 5 | Laurinsäure | 350 | 215 | 8 | 82-84 | 99,4 | 99,6 | 0 |
| 6 | Oktansäure | 180 | 150 | 6 | 62-68 | 97,3 | 97,6 | 0 |
| 7 | Stearinsäure | 285 | 125 | 5 | 74-82 | 99,7 | 99,7 | 0 |

## Ansprüche

1. Verfahren zur Herstellung von Säurechloriden durch Umsetzung von Carbonsäuren oder deren Anhydriden mit Phosgen in Gegenwart eines Dialkylformamids, dadurch gekennzeichnet, daß man als Dialkylformamid Diisobutylformamid verwendet.

2. Verfahren nach Anspruch 1, dadurch gekennzeichnet, daß man das Diisobutylformamid in einer Menge von 0,1 bis 0,4, bezogen auf die Carbonsäure, anwendet.

3. Verfahren nach Anspruch 1, dadurch gekennzeichnet, daß man die Umsetzung bei Temperaturen von 30 bis 120 °C vornimmt.

**Claims**

1. A process for the production of acid chlorides by reacting carboxylic acids or their anhydrides with phosgene in the presence of a dialkyl formamide, wherein diisobutyl formamide is used as the dialkyl formamide.

2. A process as claimed in claim 1, wherein the diisobutyl formamide is used in an amount of from 0.1 to 0.4 mole-%, based on the carboxylic acid.

3. A process as claimed in claim 1, wherein the reaction is carried out at a temperature of from 30° to 120 °C.

**Revendications**

1. Procédé de préparation de chlorures d'acides par réaction d'acides carboxyliques ou de leurs anhydrides avec le phosgène en présence d'un dialkylformamide, caractérisé en ce qu'on utilise en tant que dialkylformamide le diisobutylformamide.

2. Procédé selon la revendication 1, caractérisé en ce qu'on utilise le diisobutylformamide en quantité de 0,1 à 0,4 par rapport à l'acide carboxylique.

3. Procédé selon la revendication 1, caractérisé en ce que la réaction est effectuée à des températures de 30 à 120 °C.

4